(19) Europäisches Patentamt
European Patent Office
Office européen des brevets

(11) Publication number: **0 127 124**
**A2**

# (12) EUROPEAN PATENT APPLICATION

(21) Application number: **84105887.8**

(22) Date of filing: **23.05.84**

(51) Int. Cl.³: **C 07 C 103/52**
A 61 K 37/02
//C07C143/78, C07C143/70

(30) Priority: **23.05.83 US 497334**

(43) Date of publication of application:
**05.12.84 Bulletin 84/49**

(84) Designated Contracting States:
**AT BE CH DE FR GB IT LI LU NL SE**

(71) Applicant: **USV PHARMACEUTICAL CORPORATION**
**1 Scarsdale Road**
**Tuckahoe New York(US)**

(72) Inventor: **Piwinski, John J.**
**271 King Street**
**Port Chester New York(US)**

(72) Inventor: **Suh, John T.**
**59 Stanwich Road**
**Greenwich Connecticut(US)**

(72) Inventor: **Menard, Paul R.**
**1 Consulate Drive**
**Tuckahoe New York(US)**

(72) Inventor: **Jones, Howard**
**79 Briarcliff Woods**
**Ossining New York(US)**

(72) Inventor: **Neiss, Edward S.**
**200 Charter Oak Drive**
**New Canaan Connecticut(US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath**
**Maximilianstrasse 58**
**D-8000 München 22(DE)**

(54) **Compounds for treating hypertension.**

(57) Compounds of the formula

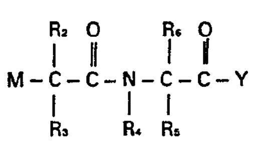

and their pharmaceutically acceptable salts, wherein the substituents are as defined herein, having antihypertensive activity.

EP 0 127 124 A2

Croydon Printing Company Ltd.

# COMPOUNDS FOR TREATING HYPERTENSION

This application relates to compounds, their pharmaceutically acceptable salts, and pharmaceutical preparations made therefrom, having utility in the treatment of hypertension in subjects suffering therefrom.

Broadly stated, the present invention comprises compounds of the formula

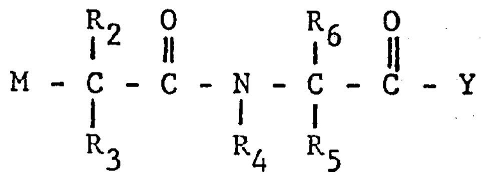 (1)

and salts thereof, especially pharmaceutically acceptable salts, wherein

Y is $-OR_1$ or $-NR(R)$;

M is X-D-, X-NH-D-, X-C(O)NH-D; $X-SO_2-D-$, X-C(O)-D-, $X-CH_2(CH_2)_nC(O)CHNH-$,
$COOR_1$

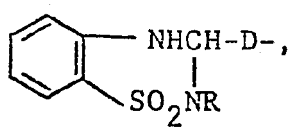

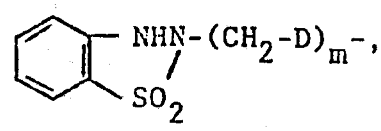

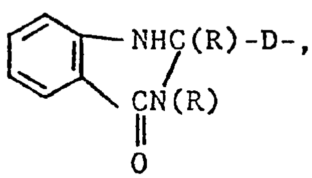 or 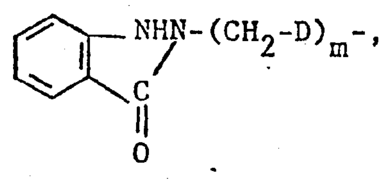

wherein D is $-(CH_2)_nN(R)-$ or $-(CH_2)_nCHNR-$, m is 0 or 1,
$COOR_1$

0127124

n is 0 to 6 inclusive, and X is a phenyl ring substituted with either two or three of the substituents: halogen, alkyl, cycloalkyl, nitroalkylamino, acyl, trifluoromethyl, nitro, cyano, -OR, -SR, -C(O)OR, -S(O)R, $-SO_2R$, -NR(R), -C(O)NR(R), $-SO_2NR(R)$, and $-NH(CH_2)_nNHR$;

R in each occurrence is independently hydrogen, alkyl, cycloalkyl, alkoxycarbonyl, acyl, aryl, aralkyl, heteroaryl-alkyl, or heteroaryl;

each of $R_1$, $R_2$, $R_3$, $R_4$, $R_5$ and $R_6$ is R, acylamino, alkylamino, alkylaminoalkyl, fused aryl-cycloalkyl, fused aryl-cycloalkyl-alkyl, or fused polycyclic aryl, and

$R_4$ and $R_5$ when taken together form an alkylene bridge containing up to 6 carbon atoms which is optionally substituted with alkyl or fused with an aryl ring,

wherein the alkyl groups and moieties may include as substituents hydroxy, nitro, acyloxy, aryl, alkoxy, aryloxy, amino, mono- or dialkylamino, acylamino, mercapto, mercaptoalkyl, or alkylthio; the cycloalkyl rings may include one or more hetero atoms, may be saturated or unsaturated, and may include as substituents alkyl, hydroxy, alkylamino, or nitro; and the aryl rings other than the phenyl moiety of M may contain one or more hetero atoms and may include as substituents carboxy, cyano, lower alkoxy-carbonyl, alkyl, aryl, aryloxy, aralkyl, acylamino, hydroxy, alkoxy, hydroxyalkyl, halo, trifluoromethyl, mercapto, alkylthio, mercaptoalkyl, amino, alkylamino, aminoalkyl, nitro, methylenedioxy, or sulfamoyl; and

wherein the alkyl groups and alkyl moieties contain up to 9 carbon atoms, the cycloalkyl rings contain 3 to 12 carbon atoms, and the aryl rings and moieties contain up to

12 carbon atoms.

Preferred compounds of the present invention are those of the general formula given above in which Y is hydroxy, benzyloxy, or lower alkoxy; $R_1$, $R_2$, $R_3$, $R_4$, $R_5$ and $R_6$ are each hydrogen, alkyl, aryl, aralkyl, cycloalkyl, or w-amino ("omega-amino") alkyl wherein the amino is mono- or disubstituted with hydrogen, alkyl, aryl, or aralkyl, or is incorporated in a saturated or unsaturated one- or two-ring heterocyclic moiety containing preferably up to 12 atoms in the ring.

The alkyl groups per se and the alkyl moieties in alkoxy, aralkyl, cycloalkyl, aminoalkyl, and the like, may be straight-chained or branched and preferably contain from 1 to 9 carbon atoms. Such groups include methyl, ethyl, propyl, isopropyl, butyl, isobutyl, tertiary-butyl, amyl, iso-amyl, hexyl, octyl, and the like. Preferably the alkyl groups are lower alkyl, which term shall refer to alkyl groups containing from 1 to 6 carbon atoms, straight-chained or branched.

The preferred substituents on the alkyl groups and moieties include hydroxy, alkoxy, hydroxy-lower alkyl, amino, acylamino, alkylamino, dialkylamino, mercapto, mercaptoalkyl, nitro, and the like.

Preferred structures for the M group include those in which R is hydrogen or lower alkyl, and n is 0, 1, 2, 3 or 4, including those with the structure X-C(O)NH-. In notation such as -NR(R), in which "R" is written twice, we intend to indicate that each R can independently be any of the possible substituents listed above for R. The moiety M is preferably connected to the rest of the molecule by a non-labile bond, so that the molecule (1) resists cleavage in the stomach.

The molecule is thus intact when it enters the blood, which can enhance the therapeutic value of the compound and can reduce undesirable side-effects. Preferred substituents for the phenyl moiety of M. by which is meant the phenyl ring X and the phenyl moiety of the polycyclic structures depicted above, include nitro, halogen, lower alkyl, carboxy-lower alkoxy, phenoxy, and hydroxy, sulfamoyl which is optionally substituted with alkyl, acyl, acylaminoalkyl, aralkyl, and heteroarylalkyl; and amino which is optionally substituted with alkyl, phenyl, aralkyl, heteroaryl-lower alkyl, nitro-alkyl, alkyl-carbonyl, and acyl-aminoalkyl (e.g. $CH_3C(O)NH(CH_2)_{2-4}-$).

In a preferred embodiment of the invention, $R_4$ and $R_5$ can be linked to form an alkylene (i.e. $-(CH_2)_i-$) bridge 3, 4, 5 or 6 carbon atoms in length. In this embodiment, the alkylene bridge preferably forms a proline ring with the nitrogen and carbon atoms to which $R_4$ and $R_5$ are respectively attached. The alkylene bridge is optionally substituted with lower alkyl. The alkylene bridge can also be fused with an aryl ring; a preferred example is where $R_4$ and $R_5$ form a tetrahydroisoquinoline ring, i.e.;

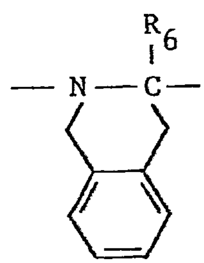

The cycloalkyl groups and moieties are saturated or unsaturated and preferably contain 3 to 9 carbon atoms. The cycloalkyl, aryl, polycycloalkyl, fused polycyclic aryl, and fused aryl-cycloalkyl structures can also contain one or

0127124

more, preferably one to three, hetero atoms, thereby forming a hetero-ring.

The preferred cyclic and polycyclic ring structures, aryl and otherwise, that $R_1$, $R_2$, $R_3$, $R_4$, $R_5$, and $R_6$ can be contain up to 20 carbon atoms and include such radicals as cyclopropyl, cyclobutyl, cyclopentyl, cyclohexyl, cycloheptyl, adamantyl, norbornyl, penyl, tolyl, benzyl, penethyl, indolyl, indolino, dimethoxyphenyl, hydroxybenzyl, indanyl, naphthyl, tetrahydronaphthyl, decanhydronaphthyl, pyridyl, quinolyl, guanidino, pyrrolinyl, piperidinyl, pyrrolidyl, pyrrolyl, morpholinyl, furyl, furfuryl, tetrahydrofurfuryl, benzimidazolyl, thienyl, imidazolyl, imidazolidinyl, quinolyl, tetrahydroquinolyl, isoquinolyl, tetrahydroisoquinolyl, benzothiadiazinyl, and the like, specifically including all isomers of structures having more than one isomer. Preferred substituents on the ring structures other than the phenyl moiety of M include hydroxy, lower alkyl, lower alkoxy, aryl, aryloxy, aralkyl, alkylamino, dialkylamino, carboxy, carboalkoxy, cyano, mercapto, amino, mercaptoalkyl, halo, trifluoromethyl, sulfamoyl, substituted sulfamoyl, nitro, and the like.

The halo groups include fluoro, chloro, bromo and iodo. Preferred hetero atoms are S, O, and N. Preferred acyl groups are lower alkyl-carbonyl and aryl-carbonyl, specifically benzoyl.

Preferred compounds are those in which at least one, and more preferably both of $R_2$ and $R_6$ are hydrogen, and $R_3$ is hydrogen, lower alkyl, or amino-lower alkyl. Preferably, where M includes two nitrogen atoms as a part of chain, the nitrogens are separated by at least two carbon

atoms.

The particularly preferred compounds of the invention are those in which at least one sulfamyl group is present; these compounds also possess significant diuretic activity in addition to ACE inhibition. Of particular value are those compounds which include sulfamyl-substituted phenyl groups and benzothiadiazine-1, 1-dioxides and the 3,4-dihydro derivatives thereof.

It will be understood by those skilled in the art that the carbons to which $R_2$ and $R_6$ are attached can be asymmetric centers, such that the inventive compounds may exist in (R,R), (R,S), (S,R), and (S,S) forms. The M group can also contain a carbon atom which is an asymmetric center. Individual isomers and diastereo-isomeric mixtures of said forms are within the scope of the invention. The preferred forms have (S,S) or (S,S,S) configuration.

The products of the present invention can be prepared by amide forming reaction of an acid of formula 1A or amide forming derivative thereof

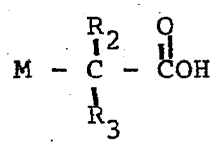

(1A)

with a compound of formula 1(B)

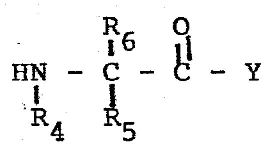

(1B)

Alternatively, the present compounds can be formed by condensation of a compound of formula 1C

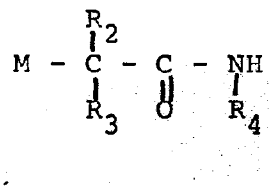

(1C)

with a compound of formula 1D

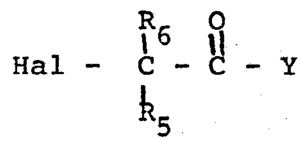

(1D)

in which Hal is halogen, under conditions of hydrogen halide removal.

Alternatively, compounds of formula I can be prepared by reaction of an amino compound of formula (1E):

$$M - H \qquad (1E)$$

with a halogen compound of formula (1F):

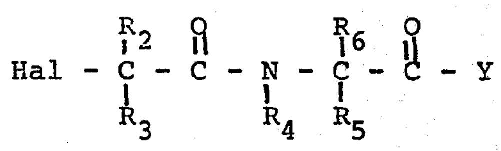

(1F)

through dehydrohalogenation reaction.

Those compounds in which M is X-C(O)NH-D- can be prepared by acylation of a compound of formula (1G)

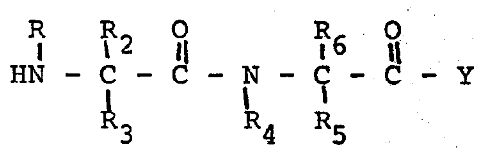

(1G)

with an acid, or acylating derivative, of the formula (1H):

$$X - CO_2H \qquad (1H)$$

In each of the foregoing reactions, reactive groups such as -C(O)-Y should be in the ester form to prevent unwanted bond formation at the Y position. The preferred esters are ethyl, t-butyl and benzyl esters. Nitrogen atoms can be protected with the usual groups used for this purpose, e.g. the benzyloxycarbonyl group or 2,2,2-frichloro-ethoxycarbonyl group.

The amide forming conditions referred to herein, involve the use of known derivatives of the described acids, such as the acyl halides, anhydrides, mixed anhydrides, lower alkyl esters, carbodiimides, carbonyl diimidazoles, and the like. The reactions are carried out in organic solvents such acetonitrile, tetrahydrofuran, dioxane, acetic acid, methylene chloride, ethylene chloride and similar such solvents. The amide forming reaction will occur at room temperature or at elevated temperature. The use of elevated temperature is for convenience in that it permits somewhat shortened reaction periods. Temperatures ranging from 0°C. up to the reflux temperature of the reaction system can be used. As a further convenience the amide forming reaction can be effected in the presence of a base such as tertiary organic amines, e.g., trimethylamine, pyridine, picolines and the like, particularly where hydrogen halide is formed by the amide-forming reaction, e.g., acyl halide and amino compound. Of course, in those reactions where hydrogen halide is produced, any of the commonly used hydrogen halide acceptors can also be used.

In the condensation of an alpha haloacid derivative, similar reaction conditions, solvents and hydrogen halide acceptors can be used as for amide formation.

Various substituents on the present new compounds can be present in the starting compounds or added after formation of the amide products by the known methods of substitution or conversion reactions. Thus, the nitro group can be added to the final product by nitration of the aromatic ring and the nitro group converted to other groups, such as amino by reduction, and halo by diazotization of the amino group and replacement of the diazo group. Other reactions can be effected on the formed amide product. Amino groups can be alkylated to form mono and dialkylamino groups, mercapto and hydroxy groups can be alkylated to form corresponding ethers. Thus, substitution or alteration reactions can be employed to provide a variety of substituents throughout the molecule of the final products. Of course, reactive groups where present should be protected by suitable blocking groups during any of the aforesaid reactions particularly the condensation reactions to form the amide linkages.

If the product of any of the above routes has one or more protecting groups, those groups are now removed, for instance with trifluoroacetic acid, or zinc in acetic acid, or basic hydrolysis, or other means appropriate for the particular protecting group chosen. If desired, ester groups in the Y position are converted to the free acid with a commonly known reagent such as HCl or NaOH.

Each of the above reactions proceeds in a straight-forward manner in a suitable solvent at temperatures ranging from 0°C to 150°C.

The products are sometimes obtained as a mixture of diasteroisomers which can be separated by standard methods of fractional crystallization or chromatography.

The compounds of this invention form acid salts with various inorganic and organic acids which are also within the scope of the invention. The pharmaceutically-acceptable acid addition salts of the compounds of the present invention may be prepared by conventional reactions by reacting the free amino acid or amino ester with an appropriate acid providing the desired anion, either in a solvent or medium in which the salt is insoluble, or in water and removing the water by freezedrying. The salts of strong acids are preferred. As exemplary, but not limiting, of pharmaceutically-acceptable acid salts are the salts of hydrochloric, hydrobromic, sulfuric, nitric, acetic, fumaric, malic, maleic and citric acids.

The compounds of this invention also form salts with bases. Suitable such salts include salts with alkali and alkaline earth metals such as sodium, potassium, magnesium and calcium, as well as iron, and salts with ammonia and amines, and quaternary ammonium salts.

The action of the enzyme renin or angiotensinogen, a pseudoglobulin in blood plasma, produces the decapeptide angiotensin I. Angiotensin I is converted by angiotensin converting enzyme (ACE) to the octapeptide angiotensin II. The latter is an active pressor substance which has been implicated as the causative agent in various forms of hypertension in various mammalian species, e.g., rats and dogs. The compounds within the scope of this invention which intervene in the renin -to- angiotensin I -to- angiotensin II

sequence inhibit angiotensin I converting enzyme and therefore are useful in reducing or relieving hypertension.

Furthermore, the compounds within the scope of the present invention which possess diuretic activity promote relief from hypertension by promoting diuresis, and consequently have utility in treating congestive heart failure. Compounds within the scope of this invention can also simultaneously possess ACE inhibitory and diuretic activity, which is particularly unexpected in view of the fact that such simultaneous activity cannot be predicted from prior art compounds. Thus by the administration of a composition containing one or a combination of compounds of formula (1) or pharmaceutically-acceptable salts thereof, hypertension in the species of mammal suffering therefrom is alleviated. A single dose, or preferably two or four divided daily doses, provided on a basis of about 0.1 to 100 mg per kilogram per day, preferably about 1 to 50 mg per kilogram per day, is appropriate to reduce blood pressure. The substance is preferably administered orally, but a parenteral route such as subcutaneously, intramuscularly, intravenously or intraperitonealy can also be employed.

The compounds of the invention can be utilized to achieve the reduction of blood pressure by formulating one or more of them in compositions such as tablets, capsules or elixirs for oral administration or in sterile solutions or suspensions for parenteral administration. About 10 to 500 mg of a compound or mixture of compounds of formula (1) or physiologically acceptable salt(s) thereof is compounded with a physiologically acceptable vehicle, carrier, excipient, binder, preservative, stabilizer, flavor, etc., in a unit dosage form as called for by accepted pharmaceutical pracice. The amount of active substance in these compositions or

preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as dicalcium phosphate; a disintegrating agent such as corn starch, potato starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate, and the like. Buffers, preservatives, antioxidants and the like can be incorporated as required. Specific embodiments of the invention are illustrated in the following examples.

EXAMPLE I

To a mixture containing 15.5 gm (0.075 mol) of N,N'-dicyclohexylcarbodiimide in 200 ml of methylene chloride at -5°C was added dropwise a solution containing 16.3 gm

(0.073 mol) of N-benzyloxycarbonyl-L-alanine and 12.5 gm (0.073 mol) of L-proline t-butyl ester in 250 ml of methylene chloride. The reaction mixture was stirred for 3 hours, after which it was filtered. The filtrate was washed twice with 0.05 M aqueous citric acid solution, once with water, and three times with saturated aqueous sodium bicarbonate. The organic phase was dried over magnesium sulfate, filtered, concentrated in vacuo, and chromatographed via HPLC (Water's 500; 33% EtOAc in hexanes) to afford 15.6 gm (57%) of N-[N-benzyloxycarbonyl-L-alanyl]-L-proline t-butyl ester as an oil.

A mixture of 8.30 gm (22.0 mmol) of N-[N-benzyloxy-carbonyl-L-alanyl]-L-proline t-butyl ester and 1.27 gm of 10% palladium on activated carbon in 250 ml of absolute ethanol was hydrogenated at 55 psi for 2 hours. The mixture was filtered through celite and concentrated in vacuo to yield 5.27 gm (99%) of N-[L-alanyl]-L-proline t-butyl ester as an oil.

A mixture of 50.0 gm (0.151 mol) of 4-chloro-N-furfuryl-5-sulfamoylanthranilic acid in 1.0 liter of 1.2N aqueous hydrochloric acid was refluxed for 1 hour. The reaction mixture was filtered hot and the filtrate gradually cooled to room temperature. The precipitated solid was filtered off and recrystallized twice by dissolving it in 1N aqueous sodium hydroxide followed by filtration and acidification with 1N aqueous hydrochloric acid. Trituration of the product in boiling ether afforded 17.0 gm (45%) of 2-amino-4-chloro-5-sulfamoylbenzoic acid: mp 259-261°C.

To a solution of 1.9 gm (7.6 mmol) of 2-amino-4-chloro-5-sulfamoylbenzoic acid, 2.0 gm (8.3 mmol) of N-[L-alanyl]-L-proline t-butyl ester, and 2.7 gm (17.6

mmol) of 1-hydroxybenzotriazole hydrate in 80 ml of dry tetrahydrofuran at 0°C and under an atmosphere of nitrogen was added dropwise a solution of 1.73 gm (8.39 mmol) of N, N'-dicyclohexylcarbodiimide in 25 ml of dry tetrahydrofuran. The reaction mixture was slowly allowed to warm to room temperature. After 18 hours the mixture was filtered and the filtrate was concentrated _in vacuo_. The residue was taken up in ethyl acetate and washed twice with 10% aqueous sodium dihydrogen phosphate and once with a saturated solution of sodium bicarbonate. The organic portion was dried over magnesium sulfate, filtered and concentrated _in vacuo_. The product was recrystallized from ethyl acetate to afford 1.40 gm (36%) of N-[N-(2-amino-4-chloro-5-sulfamoylbenzoyl)-L-alanyl]-L-proline t-butyl ester (I-A).

A mixture of 605 mg (1.27 mmol) of N-[N-(2-amino-4-chloro-5-sulfamoylbenzoyl)-L-alanyl]-L-proline t-butyl ester and 5.0 ml (65 mmol) of trifluoroacetic acid in 20 ml of methylene chloride was stirred at room temperature for 3 hours. The reaction mixture was concentrated _in vacuo_ and the residue triturated with ether to afford 520 mg (98%) of N-[N-(2-amino-4-chloro-5-sulfamoylbenzoyl)-L-alanyl]-L-proline (I-B). This product could be recrystallized from chloroform/methanol/acetic acid (85/15/5) to give N-[N-(2-amino-4-chloro-5-sulfamoylbenzoyl)-L-alanyl]-L-proline as its hemihydrate: mp 185-187°C.

EXAMPLE II

A mixture of 8.39 gm (31.1 mmol) of 2,4-dichloro-5-sulfamoylbenzoic acid and 20.0 ml (183 mmol) of benzylamine was heated at 130°C for 2 hours. The reaction mixture was poured into a solution of 10% aqueous acetic acid at 0°C. The precipitated oil was removed and dissolved in 1.0N aqueous sodium hydroxide (pH 9). After filtration the product was reprecipitated by acidification of the solution (pH 1) with concentrated hydrochloric acid. The solid product was collected, dissolved in tetrahydrofuran, dried over magnesium sulfate/charcoal, filtered, and concentrated *in vacuo*. The product was recrystallized from hot tetrahydrofuran/hexanes to yield 3.20 gm (30%) of 2-benzylamino-4-chloro-5-sulfamoylbenzoic acid: mp 241-243°C (lit 238-240°C).

To a solution of 3.47 gm (10.2 mmol) of 2-benzylamino-4-chloro-5-sulfamoylbenzoic acid, 2.66 gm (11.0 mmol) of N-[L-alanyl]-L-proline t-butyl ester, and 3.72 gm (24.3 mmol) of 1-hydroxybenzotriazole hydrate in 40 ml of dry tetrahydrofuran at 0°C and under an atmosphere of nitrogen was added dropwise over a 5 minute period a solution of 2.33 gm (11.3 mmol) of N,N'-dicyclohexylcarbodiimide in 10 ml of dry tetrahydrofuran. The reaction mixture was slowly allowed to warm to room temperature. After 18.5 hours the mixture was filtered and concentrated *in vacuo*. The residue was taken up in ethyl acetate and washed three times with 10% aqueous sodium dihydrogen phosphate, twice with a saturated solution of sodium bicarbonate, twice with water, and once with brine. The organic portion was dried over magnesium sulfate, filtered, concentrated *in vacuo*, and chromatographed via HPLC twice [Water's 500; 50% EtOAc in hexanes (k'=2.0);

then 25% EtOAc in $CH_2Cl_2$ (k'=4.6)] to afford 2.15 gm (37%) of N-[N-(2-benzylamino-4-chloro-5-sulfamoylbenzoyl)-L-alanyl]-L-proline t-butyl ester (II-A): mp 183-185°C.

To a mixture of 1.30 gm (2.30 mmol) of N-[N-(2-benzylamino-4-chloro-5-sulfamoylbenzoyl)-L-alanyl]-L--proline t-butyl ester in 50 ml of dry methylene chloride at room temperature and under an atmosphere of nitrogen was added dropwise over a period of 15 minutes 10.0 ml (130 mmol) of trifluoroacetic acid. After 4.5 hours the mixture was concentrated in vacuo and the residue triturated with ether. The white solid was recrystallized from hot tetrahydrofuran/hexanes to yield 870 mg (73%) of N-[N-(2-benzylamino-4-chloro-5-sulfamoylbenzoyl)-L-alanyl]-L-proline (II-B) as its hemihydrate: m.p. 160-163°C.

## EXAMPLE III

A mixture of 46.8 gm (0.173 mol) of 2,4-dichloro-5-sulfamoylbenzoic acid in 400 ml of 5N ethanolic hydrogen chloride solution was stirred at room temperature for 64 hours. The reaction mixture was concentrated in vacuo and the product recrystallized from hot ether to yield 38.5 gms (75%) of ethyl 2,4-dichloro-5-sulfamoylbenzoate: mp 119-122°C.

A mixture containing 14.1 gm (58.2 mmol) of N-[L-alanyl]-L-proline t-butyl ester and 11.0 gm (36.9 mmol) of ethyl 2,4-dichloro-5-sulfamoylbenzoate in 75 ml of tetrahydrofuran was refluxed for 38 hours. The reaction mixture was concentrated in vacuo and the product chromatographed by HPLC [Water's 500; 95% ether in hexanes]. Upon concentration in vacuo of the various fractions white crystals precipitated which were filtered off and checked for purity by TLC [ether]. Two major products were isolated

separately and identified as N-[N-(4-ethoxycarbonyl-5-chloro-2-sulfamoylphenyl)-L-alanyl]-L-proline t-butyl ester (III-A) (955 mg, 5.1%, mp 194-195°C, $R_f$=0.51) and N-[N-(2-ethoxycarbonyl-5-chloro-4-sulfamoylphenyl)-L-alanyl]-L-proline t-butyl ester (III-B) (1.981 gms, 10.6%, mp 208-211°C, $R_f$=0.38).

A mixture containing 404 mg (0.802 mmol) of N-[N-(2-ethoxycarbonyl-5-chloro-4-sulfamoylphenyl)-L-alanyl]-L-proline t-butyl ester and 1.50 ml (19.5 mmol) of trifluoroacetic acid in 10 ml of methylene chloride was stirred at room temperature for 18 hours. The reaction mixture was concentrated *in vacuo* and the residue triturated with ether to give a solid which was chromatographed via HPLC [Water's 500; k'=2.1; Ether/$CH_2Cl_2$/EtOH/AcOH(50/46.5/2.5/2)] to yield 305 mg (85%) of N-[N-(2-ethoxycarbonyl-5-chloro-4-sulfamoylphenyl)-L-alanyl]-Lproline (III-C): mp 153-157°C.

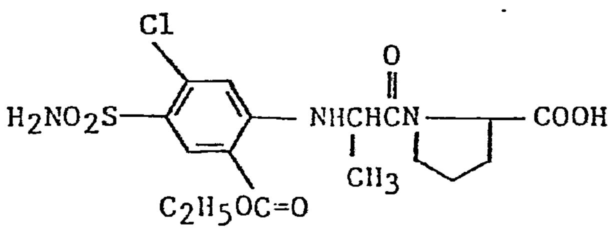

(III-C)

EXAMPLE IV

A mixture containing 566 mg (1.12 mmol) of N-[N-(4-ethoxycarbonyl-5-chloro-2-sulfamoylphenyl)-L-alanyl]--L-proline t-butyl ester (III-A) in 10 ml of 4N hydrogen chloride in dioxane at room temperature and under an atmosphere of nitrogen was stirred for 16.5 hours. The reaction mixture was filtered and concentrated *in vacuo* and the residue was triturated with ether/hexanes. The product was filtered off and dried (T about 40°C) under vacuum to give 465 mg (93%) of N-[N-(4-ethoxycarbonyl-5-chloro-2-sulfamoylphenyl)-L-alanyl]-L-proline IV-A) as a white solid:

mp 113-116°C.

EXAMPLE V

Para-chlorobenzoic acid (281 g, 1.79 mol) was added portionwise with stirring to chlorosulfonic acid (1051 g, 600 ml, 9.02 mol) cooled to 0°C. The resulting mixture was heated at 160°C for 2 hours, then cooled and poured very slowly over crushed ice (4 liters). The precipitate was filtered and washed with water; 356.3 g (78%) of 4-chloro-3-chlorosulfonylbenzoic acid were obtained.

A mixture of 4-chloro-3-chlorosulfonylbenzoic acid (150 g, 0.58 mol) in concentrated sulfuric acid (816 ml) was added over one hour to conc. sulfuric acid (324 ml)/conc. nitric acid (201 ml). The mixture was heated at 80-90°C for six hours, then cooled and poured slowly over crushed ice (2 liters). The white precipitate (147.7 g, 85%) of 4-chloro-5-chlorosulfonyl-3-nitrobenzoic acid was collected.

4-Chloro-5-chlorosulfonyl-3-nitrobenzoic acid (100 g, 0.33 mol) was added to condensed anhydrous ammonia (250 ml) at -78°C. The mixture was stirred for four hours at -78°C, then the ammonia was allowed to evaporate at room temperature. The residue was suspended in water and the mixture was acidified to pH 1 with concentrated hydrochloric acid. The mixture was heated to boiling and filtered; on cooling, a precipitate formed and was collected. Yield was 51.2 g (55%) of 4-chloro-3-nitro-5-sulfamoylbenzoic acid (V-A).

4-Chloro-3-nitro-5-sulfamoylbenzoic acid (2.0 g, 7.1 mmol) and N-[L-alanyl]-L-proline t-butyl ester (2.0 g, 8.3 mmol) were combined in acetonitrile (40 ml) and cooled to 0°C. To this was added dropwise a solution of N,N'-dicyclohexylcarbodiimide (1.6 g, 7.8 mmol) in

acetonitrile (20 ml). After stirring for 3 hours at 0°C, the mixture was filtered and the filtrate was added to 10% sodium dihydrogen phosphate solution (200 ml) and stirred for 5 minutes. The precipitate was collected and washed with saturated sodium bicarbonate solution and water; 2.1 g (58%) of N-[N-(4-chloro-3-nitro-5-sulfamoylbenzoyl)-L-alanyl]-L-proline t-butyl ester (V-B) was thus obtained. Recrystallization from water/ethanol gave a product of higher purity.

A solution of N-[N-(4-chloro-3-nitro-5-sulfamoylbenzoyl)-L-alanyl]-L-proline t-butyl ester (4.1 g, 8.1 mmol) in dioxane saturated with HCl (40 ml) was prepared. After 3.5 hours the product was filtered, washed with water and recrystallized from ethanol. Yield was 2.4 g (67%) of N-[N-(4-chloro-3-nitro-5-sulfamoylbenzoyl)-L-alanyl]-L-proline (V-C).

EXAMPLE VI

A suspension of 3.9 g (7.7 mmol) of N-[N-(4-chloro-3-nitro-5-sulfamoylbenzoyl)-L-alanyl]-L-proline t-butyl ester (compound V-B) and Raney nickel catalyst in ethanol (150 ml) was hydrogenated at 40-50 psi hydrogen for 2 hours in a Parr apparatus. The mixture was filtered hot through celite; on cooling the filtrate deposited white needles. Yield was 2.6 g (70%) of N-[N-(3-amino-4-chloro-5-sulfamoylbenzoyl)-L-alanyl]-L-proline t-butyl ester (VI-A).

N-[N-(3-amino-4-chloro-5-sulfamoylbenzoyl)-L-alanyl]-L-proline t-butyl ester (2.1 g, 4.4 mmol) was combined with 4N HCl/dioxane (25 ml) and heated to 60°C for 2 hours. The mixture was concentrated *in vacuo* to provide a white hygroscopic solid, N-[N-(3-amino-4-chloro-5-sulfamoylbenzoyl)-L-alanyl]-L-proline (VI-B).

EXAMPLE VII

A mixture of 1.9 g (4.5 mmol) of N-[N-(3-amino-4-chloro-5-sulfamoylbenzoyl)-L-alanyl]-L-proline (compound VI-B), 2-nitroethyl acetate (0.60 g, 4.5 mmol), acetic acid (0.30 g, 4.5 mmol), sodium acetate trihydrate (0.60 g, 4.5 mmol) and water (2.0 ml) was slowly heated to 56°C and held there 5 minutes. After cooling, the precipitate was filtered and washed with water; 1.6 g (73%) of N-[N-(4-chloro-3-(2-nitroethylamino)-5-sulfamoylbenzoyl]-L-alanyl]-L-proline (VII-A) was thus obtained.

EXAMPLE VIII

A mixture of 14 g (0.050 mol) of 4-chloro-3-nitro-5-sulfamoylbenzoic acid, (compound V-A), sodium bicarbonate (17 g, 0.20 mol), phenol (10 g, 0.11 mol) and water (100 ml) was heated at 85°C for 16 hours. On cooling, a precipitate formed and was collected. This material was dissolved in hot water (150 ml) and the solution acidified (conc. hydrochloric acid). The precipitate was filtered, washed with water and recrystallized from water/ethanol to give 6.0 g (35%) of 3-nitro-4-phenoxy-5-sulfamoylbenzoic acid.

3-Nitro-4-phenoxy-5-sulfamoylbenzoic acid (3.0 g, 8.9 mmol) and N-[L-alanyl]-L-proline t-butyl ester (2.4 g, 10 mmol) were combined in acetonitrile (100 ml) and brought to reflux. When dissolution had occurred, N,N'-dicyclohexylcarbodiimide (2.1 g, 10 mmol) in acetonitrile (20 ml) was added dropwise. After refluxing an additional 3 hours, the mixture was cooled to 60-70°C, filtered and quickly refiltered. The filtrate was cooled to 0°C and the precipitate collected and washed with water. Yield was 2.1 g, (42%) of N-[N-(3-nitro-4-phenoxy-5-sulfamoylbenzoyl)-L-alanyl]-L-proline t-butyl ester (VIII-A).

N-N-(3-Nitro-4-phenoxy-5-sulfamoylbenzoyl)-L-alanyl]-L-proline t-butyl ester (1.0 g, 1.8 mmol) was dissolved in 4N HCl/dioxane (5 ml) and allowed to stir for six days. Removal of solvent in vacuo provided the desired product, N-[N-(3-nitro-4-phenoxy-5-sulfamoylbenzoyl)-L-alanyl]-L-proline (VIII-B).

EXAMPLE IX

A mixture of 3.0 g (5.3 mmol) of N-[N-(3-nitro-4-phenoxy-5-sulfamoylbenzoyl)-L-alanyl]-L-proline t-butyl ester (compound VIII-A) and 10% palladium on carbon (1.0 g) in ethanol (200 ml) was hydrogenated on a Parr apparatus for 1.5 hours at an initial hydrogen pressure of 40-50 psi. The mixture was filtered through celite and the filtrate concentrated in vacuo to give 1.9 g (68%) of N-[N-(3-amino-4-phenoxy-5-sulfamoylbenzoyl)-L-alanyl]-L-proline t-butyl ester (IX-A).

N-[N-(3-Amino-4-phenoxy-5-sulfamoylbenzoyl)-L-alanyl]-L-proline t-butyl ester (0.9 g, 1.7 mmol) and 4N HCl/dioxane (20 ml) were heated to 60°C over 1 hour. Solvent was removed in vacuo and the residue was recrystallized from water. Yield was 0.40 g (50%) of N-[N-(3-amino-4-phenoxy-5-sulfamoylbenzoyl)-L-alanyl]-L-proline (IX-B).

EXAMPLE X

4-Chloro-3-sulfamoylbenzoyl chloride (4.0 g, 16 mmol), N-[L-alanyl]-L-proline t-butyl ester (4.4 g, 18 mmol) and triethylamine (1.8 g, 18 mmol) were combined in dry tetrahydrofuran (25 ml) and refluxed 3 hours. Additional triethylamine (1.8 g, 18 mmol) was introduced and reflux was continued overnight. The mixture was cooled, filtered and concentrated and the residue was washed with water and ether.

Recrystallization from water/ethanol gave 3.4 g of N-[N-(4-chloro-3-sulfamoylbenzoyl)-L-alanyl]-L-proline t-butyl ester (X-A).

N-[N-(4-Chloro-3-sulfamoylbenzoyl)-L-alanyl]-L-proline t-butyl ester (1.8 g, 3.9 mmol) was dissolved in 4N HCl/dioxane (20 ml) and stirred for 8 hours. Solvent was removed and the residue was recrystallized from water/ethanol to provide 1.6 g (100%) of N-[N-(4-chloro-3-sulfamoylbenzoyl)-L-alanyl]-L- proline (X-B).

WHAT WE CLAIM IS:

1. Compounds having antihypertensive activity of the formula

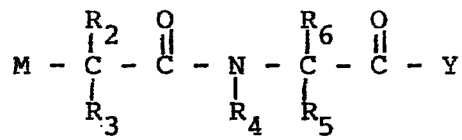

and salts thereof; wherein

Y is $-OR_1$ or $-NR(R)$;

M is X-D-, X-NH-D-, X-C(O)NH-D-, $X-SO_2-D-$, X-C(O)-D-, $X-CH_2(CH_2)_nC(O)CHNH-$,
$COOR_1$

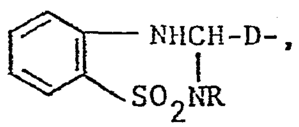

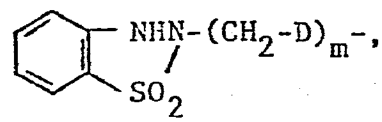

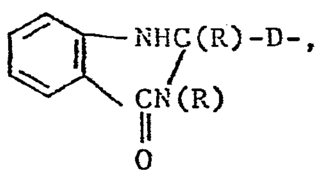 or 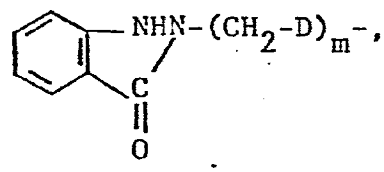

wherein D is $-(CH_2)_nN(R)-$ or $-(CH_2)_nCHNH$, m is 0 or 1,
$COOR_1$

n is 0 to 6 inclusive, and X is a phenyl ring with either two or three of the substituents halogen, alkyl, cycloalkyl, nitroalkylamino, acyl, trifluoromethyl, nitro, cyano, -OR, -SR, -C(O)OR, -S(O)R, $-SO_2R$, -NR(R), -C(O)NR(R), $-SO_2NR(R)$, and $-NH(CH_2)_nNHR$:

R in each occurrence is independently hydrogen, alkyl, cycloalkyl, alkoxycarbonyl, acyl, aryl, aralkyl, heteroaryl-alkyl, or heteroaryl;

each of $R_1$, $R_2$, $R_3$, $R_4$, $R_5$, and $R_6$ is independently selected from the group consisting of R, acylamino, alkylamino, fused aryl-cycloalkyl, fused aryl-cycloalkyl-alkyl, or fused polycyclic aryl, and

$R_4$ and $R_5$ when taken together form an alkylene bridge containing up to 6 carbon atoms which is optionally substituted with alkyl or fused with an aryl ring,

wherein the alkyl groups and moieties may include as substituents hydroxy, nitro, acyloxy, aryl, alkoxy, aryloxy, amino, mono- or dialkylamino, acylamino, mercapto, mercaptoalkyl, or alkylthio; the cycloalkyl rings may include ne or more hetero atoms, may be saturated or unsaturated, and may include as substituents alkyl, hydroxy, alkylamino, or nitro; and the aryl rings other than the phenyl moiety of M may contain one or more hetero atoms and may include as substituents carboxy, cyano, lower alkoxy-carbonyl,alkyl, aryl, aryloxy, aralkyl, acylamino, hydroxy, alkoxy, hydroxyalkyl, halo, trifluoromethyl, mercapto, alkylthio, mercaptoalkyl, amino, alkylamino, aminoalkyl, nitro, methylenedioxy, or sulfamoyl; and

wherein the alkyl groups and alkyl moieties contain up to 9 carbon atoms, the cycloalkyl rings contain 3 to 12 carbon atoms, and the aryl rings and moieties contain up to 12 carbon atoms.

2. The compound as in Claim 1 wherein there is at least one asymmetric carbon, and the substituents on each asymmetric carbon are in the "S" configuration.

3. The compound as in Claim 2 wherein at least one of $R_1$, $R_2$, and $R_6$ is hydrogen.

4. The compound as in any of Claims 1 - 3 wherein $R_4$ and $R_5$ are connected together to form a proline ring

0127124

or a tetrahydroisoquinoline ring with the nitrogen and carbon atoms to which they are respectively attached.

5. The compound as in any of Claims 1 - 4 wherein $R_1$, $R_2$, and $R_6$ are hydrogen, and Y is hydroxy, alkoxy containing up to 6 carbon atoms, or benzyloxy.

6. The compound as in any of Claims 1 - 5 wherein $R_3$ is hydrogen, alkyl, or aminoalkyl.

7. The compound as in any of Claims 1 - 6 wherein M is a substituted phenyl or benzoyl group.

8. The compound as in Claim 1 which is one of the following :

N-[N-(2-amino-4-chloro-5-sulfamoylbenzoyl)-L-alanyl]-L-proline t-butyl ester;

N-[N-(2-amino-4-chloro-5-sulfamoylbenzoyl)-L-alanyl]-L-proline;

N-[N-(2-benzylamino-4-chloro-5-sulfamoylbenzoyl)-L-alanyl]-L-proline t-butyl ester;

N-[N-(2-benzylamino-4-chloro-5-sulfamoylbenzoyl)-L-alanyl]-L-proline;

N-[N-(4-ethoxycarbonyl-5-chloro-2-sulfamoylphenyl)-L-alanyl]-L-proline t-butyl ester;

N-[N-(4-ethoxycarbonyl-5-chloro-2-sulfamoylphenyl)-L-alanyl]-L-proline;

N-[N-(2-ethoxycarbonyl-5-chloro-4-sulfamoylphenyl)-L-alanyl]-L-proline t-butyl ester;

N-[N-(2-ethoxycarbonyl-5-chloro-4-sulfamoylphenyl)-L-alanyl]-L-proline;

N-[N-(3-nitro-4-chloro-5-sulfamoylbenzoyl)-L-alanyl]-L-proline t-butyl ester;

N-[N-(3-nitro-4-chloro-5-sulfamoylbenzoyl)-L-alanyl]-L-proline;

N-[N-(3-amino-4-chloro-5-sulfamoylbenzoyl)-L-alanyl]-L-proline t-butyl ester;

N-[N-(3-amino-4-chloro-5-sulfamoylbenzoyl)-L-alanyl]-L-proline;

N-[N-[3-(2-nitroethylamino)-4-chloro-5-sulfamoylbenzoyl]-L-alanyl]-L-proline;

N-[N-(3-nitro-4-phenoxy-5-sulfamoylbenzoyl)-L-alanyl]-L-proline t-butyl ester;

N-[N-(3-nitro-4-phenoxy-5-sulfamoylbenzoyl)-L-alanyl]-L-proline;

N-[N-(3-amino-4-phenoxy-5-sulfamoylbenzoyl)-L-alanyl]-L-proline t-butyl ester;

N-[N-(3-amino-4-phenoxy-5-sulfamoylbenzoyl)-L-alanyl]-L-proline;

N-[N-(3-sulfamoyl-4-chlorobenzoyl)-L-alanyl]-L-proline t-butyl ester; and

N-[N-(3-sulfamoyl-4-chlorobenzoyl)-L-alanyl]-L-proline.